# EUROPEAN PATENT APPLICATION

(11) **EP 4 636 696 A1**
(43) Date of publication of application: **22.10.2025**
(21) Application number: 25171222.0
(22) Date of filing: 17.04.2025
(51) Int. Cl.: G06T 7/73, G06T 11/00

(54) **MEDICAL IMAGE PROCESSING DEVICE AND METHOD OF OPERATING THE SAME**

(30) Priority: 18.04.2024 JP 2024067271
(71) Applicant: FUJI-FILM Corporation, Minato-ku Tokyo 106-8620 (JP)
(72) Inventor: MACHII, Yusuke, Tokyo 1006-8620 (JP)
(74) Representative: Dehns Germany Partnerschaft mbB

(57) **Abstract**

A medical image processing device and a method of operating the same that enable a user to check an anatomical structure such as a blood vessel or a nerve in an organ during surgery without any burden by controlling display of a 3D organ model. A 3D organ model acquisition unit (20) acquires a 3D organ model. A rigid endoscope image acquisition unit (21) acquires a rigid endoscope image including an organ to be observed (45). An orientation matrix estimation unit (22) estimates an orientation matrix representing an orientation of the organ to be observed included in the rigid endoscope image in a three-dimensional coordinate system used for the 3D organ model (52a). A display control unit (23) controls display of the 3D organ model based on the orientation matrix.

## Description

### BACKGROUND OF THE INVENTION

### 1. Field of the Invention

The present disclosure relates to a medical image processing device that controls display of an organ to be observed and a 3D organ model corresponding to the organ to be observed, and a method of operating the same.

### 2. Description of the Related Art

In a current medical field, surgery is performed in an abdominal cavity using a rigid endoscope image without laparotomy. In order to realize high-quality surgery, it is important to proceed with the surgery according to a preoperative plan while checking a course of major blood vessels hidden by fat or grasping a course of blood vessels found during dissection or separation, a relationship with nearby nerves or organs, and a position of a lesion during the surgery.

As a document related to the above, JP2023-131741A discloses that a planar organ image obtained by imaging an organ and a 3D simulated image which is a 3D model that three-dimensionally simulates the organ are displayed. In addition, Augmented Reality Navigation for Stereoscopic Laparoscopic Anatomical Hepatectomy of Primary Liver Cancer: Preliminary Experience (http:pubmed.ncbi.nlm.nih.gov/33842378) discloses that liver surface 3D data is acquired from an intraoperative video and is registered with a preoperative CT model, and after the registration, a specific landmark is determined and information on the landmark is used to track an observation target.

### SUMMARY OF THE INVENTION

A 3D organ model acquired preoperatively is used to check the course of the blood vessels and their positions within the organ during the surgery. During the surgery, the 3D organ model pre-printed on paper can be referred to, or the 3D organ model can be displayed on a display. However, in the former method, an operator has to mentally compensate for a difference between the output 3D organ model and an actual intraoperative appearance of the organ, and in the latter method, it is necessary to manually operate the display to match the 3D organ model with the actual intraoperative appearance of the organ. Both methods place a burden on a user such as an operator or an assistant.

An object of an exemplary embodiment of the invention is to provide a medical image processing device and a method of operating the same that enable a user to check an anatomical structure such as a blood vessel or a nerve during surgery without any burden.

A medical image processing device according to an aspect of the exemplary embodiment comprises: a processor, in which the processor is configured to acquire a 3D organ model corresponding to an organ to be observed, which is an observation target, acquire a medical image including the organ to be observed, estimate an orientation matrix representing an orientation of the organ to be observed included in the medical image in a three-dimensional coordinate system used for the 3D organ model, and control display of the 3D organ model based on the orientation matrix.

It is also considered that the medical image and a learning model that is trained using a correct orientation matrix for the medical image as an input are used for the estimation of the orientation matrix. It is also considered that the processor is configured to perform control of rotating the 3D organ model in the three-dimensional coordinate system.

It is also considered that the processor is configured to periodically change an orientation of the 3D organ model in the three-dimensional coordinate system in a case in which a degree of overlap between structures in the organ to be observed is equal to or greater than a threshold value or in a case in which the orientation of the organ to be observed does not change for a certain period of time. It is also considered that the processor is configured to determine a degree of overlap between structures in the 3D organ model, and change an orientation of the 3D organ model such that the degree of overlap between structures falls within a specific range.

It is also considered that the processor is configured to calculate a correction amount (for example, a correction matrix, yaw, pitch, and roll) for correcting a shape difference between a predetermined reference organ and the organ to be observed and correct the orientation matrix according to the correction amount, and control the display of the 3D organ model based on the corrected orientation matrix. It is also considered that the processor is configured to estimate the orientation matrix using the 3D organ model in addition to the medical image.

It is also considered that the processor is configured to apply a process of smoothing a change in angle of the 3D organ model in a time direction to the orientation matrix. It is also considered that the estimation of the orientation matrix is performed using a plurality of the medical images obtained at different timings. It is also considered that the estimation of the orientation matrix is performed using the medical image and an orientation matrix estimated before a timing at which the estimation is performed.

It is also considered that the display of the 3D organ model is controlled in conjunction with a field of view based on the medical image. It is also considered that the processor is configured to recognize a surgical procedure from the medical image, and control ON or OFF of display control of an angle of the 3D organ model or a display update speed according to the surgical procedure.

It is also considered that the processor is configured to determine whether or not the estimation of the orientation matrix works from the medical image, and stop the display of the 3D organ model in a case in which the estimation of the orientation matrix does not work. It is also considered that the processor is configured to switch a display aspect of the 3D organ model in response to a display switching operation.

It is also considered that the processor is configured to recognize a resection progress of the organ to be observed from the organ to be observed, and switch a display aspect of the 3D organ model according to the resection progress. It is also considered that the processor is configured to, in a case in which a specific movement is recognized, activate display control of the 3D organ model using the orientation matrix. It is also considered that the processor is configured to receive a correction from a user regarding a display direction of the 3D organ model, and correction information regarding the correction is used for training the learning model or for a correction amount for correcting a shape difference between a predetermined reference organ and the organ to be observed.

It is also considered that the medical image is any one of a rigid endoscope image or an ultrasound image. It is also considered that the 3D organ model is extracted from a radiation image or an MRI image. It is also considered that the organ to be observed is any one of a liver, a kidney, a pancreas, a uterus, or a nerve. It is also considered that the processor is configured to estimate a relative positional relationship between an imaging apparatus for capturing the medical image and the organ to be observed as a display matrix representing the relative positional relationship in the three-dimensional coordinate system, and control display of the 3D organ model based on the display matrix.

A method of operating a medical image processing device according to another aspect of the exemplary embodiment comprises: via a processor, a step of acquiring a 3D organ model corresponding to an organ to be observed, which is an observation target; a step of acquiring a medical image including the organ to be observed; a step of estimating an orientation matrix representing an orientation of the organ to be observed included in the medical image in a three-dimensional coordinate system used for the 3D organ model; and a step of controlling display of the 3D organ model based on the orientation matrix.

According to the exemplary embodiments of the invention, it is possible to enable a user check an anatomical structure such as a blood vessel or a nerve during surgery without any burden by controlling display of a 3D organ model.

### BRIEF DESCRIPTION OF THE DRAWINGS

Fig. 1 is a schematic diagram of a medical image processing system.
Fig. 2 is an image diagram showing a rigid endoscope image and a 3D organ model.
Fig. 3 is an explanatory diagram showing an input and output relationship of a learning model.
Fig. 4 is an explanatory diagram showing a three-dimensional coordinate system consisting of an X-axis, a Y-axis, and a Z-axis.
Fig. 5 is an explanatory diagram showing 3D organ models before display control and after display control based on an orientation matrix.
Fig. 6 is an explanatory diagram showing periodically changing an orientation of a 3D organ model.
Figs. 7A and 7B are diagrams showing a process of smoothing a change in angle of a 3D organ model over time.
Fig. 8 is an explanatory diagram showing that three rigid endoscope images having different timings are input to the learning model.
Fig. 9 is an explanatory diagram showing that a rigid endoscope image and an orientation matrix before estimation are input to the learning model.
Fig. 10 is an explanatory diagram showing a 3D organ model of a liver in a state where blood vessels overlap each other.
Fig. 11 is a flowchart showing a method of determining a degree of overlap between structures.
Fig. 12 is an explanatory diagram showing a 3D organ model rotated at an optimal angle.
Fig. 13 is an explanatory diagram showing correction of an orientation matrix in accordance with a correction matrix.
Figs. 14A and 14B are explanatory diagrams showing a liver region of a reference liver and a liver region of an organ to be observed.
Figs. 15A and 15B are explanatory diagrams showing a centroid, a right end, and a lower end of the reference liver and a centroid, a right end, and a lower end of the organ to be observed.
Fig. 16 is an explanatory diagram showing a learning model to which a rigid endoscope image and a 3D organ model are input.
Fig. 17 is an explanatory diagram showing that an angle of a 3D organ model is rotated according to a field of view.
Fig. 18 is an explanatory diagram showing control of angle display of a 3D organ model and control of a display update speed according to a surgical procedure.
Figs. 19A and 19B are image diagrams showing a rigid endoscope image for which an orientation matrix can be estimated and a rigid endoscope image for which an orientation matrix cannot be estimated.
Fig. 20 is an explanatory diagram showing that an angle of a 3D organ model is changed by a display switching operation.
Fig. 21 is an explanatory diagram showing that an angle of a 3D organ model is changed according to a resection progress of an organ.
Figs. 22A and 22B are image diagrams showing a 3D organ model in a case in which a specific motion is recognized and a 3D organ model in a case in which a specific motion is not recognized.
Fig. 23 is an explanatory diagram showing that learning is performed based on angle correction of a 3D organ model by a user.
Fig. 24 is a flowchart showing a series of flows of display control of a 3D organ model based on an orientation matrix.

### DESCRIPTION OF THE PREFERRED EMBODIMENTS

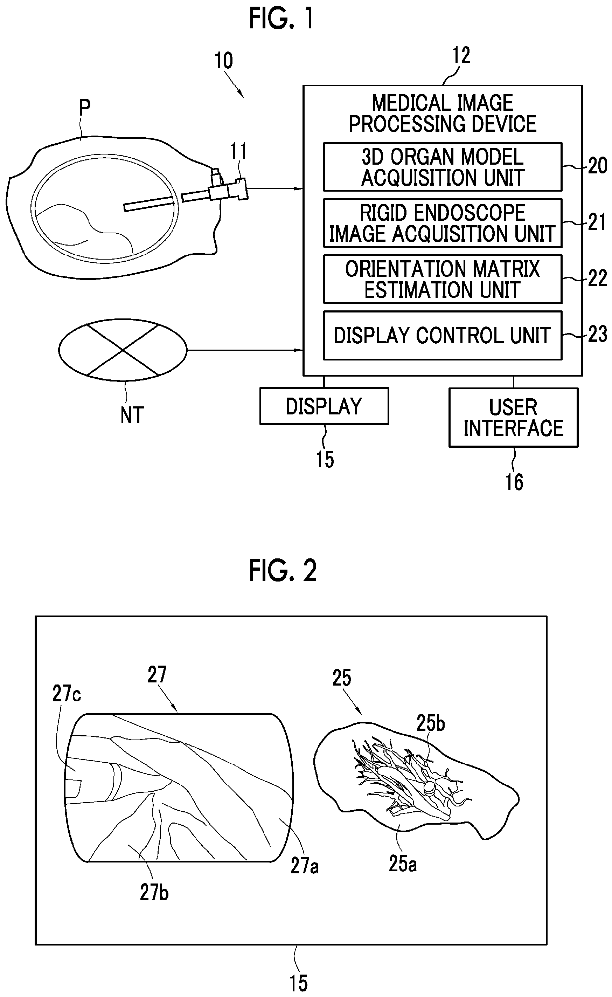

As shown in Fig. 1, a medical image processing system 10 comprises a rigid endoscope 11 and a medical image processing device 12. The rigid endoscope 11 captures an image of an inside of a body of a patient P and transmits a rigid endoscope image obtained by the imaging to the medical image processing device 12. The rigid endoscope 11 is also connected to a light source device (not shown), and illumination light from the light source device is supplied to the rigid endoscope 11.

The medical image processing device 12 is a computer such as a server, and is connected to a display 15 and a user interface 16. In addition, the medical image processing device 12 is connected to a network NT. A picture archiving and communication system (PACS) or the like is connected to the network NT, and various image data and the like from the PACS are incorporated into the medical image processing device 12 via the network NT.

In the medical image processing device 12, a program for executing various types of processing is stored in a program memory (not shown). A central control unit (not shown) configured of a processor executes the program in the program memory, so that the medical image processing device 12 implements functions of a 3D organ model acquisition unit 20, a rigid endoscope image acquisition unit 21, an orientation matrix estimation unit 22, and a display control unit 23.

The 3D organ model acquisition unit 20 acquires a 3D organ model corresponding to an organ to be observed, which is an observation target. The 3D organ model is acquired from a 3D organ model image server (not shown) or the like via the network NT. The 3D organ model is a model extracted from a radiation image such as an X-ray image or a CT image, or an MRI, and is composed of a 3D organ model 25 in which a structure such as a blood vessel is superimposed and displayed on the organ to be observed as shown in Fig. 2. Specifically, in a case in which the organ to be observed is a liver, a model in which a blood vessel 25b inside the liver is superimposed and displayed as a structure on a liver 25a is displayed on the display 15. In addition to the liver, the organ to be observed may be, for example, a kidney, a pancreas, a spleen, a uterus, or a nerve, and is not limited to the above organs and may be various other organs.

The rigid endoscope image acquisition unit 21 acquires a rigid endoscope image including the organ to be observed. The rigid endoscope image is acquired from the rigid endoscope 11. Specifically, in a case in which the organ to be observed is a liver, as shown in Fig. 2, the rigid endoscope image 27 includes not only a liver 27a but also a peripheral structure 27b, an ultrasound probe 27c that is one of various treatment tools, and the like. The rigid endoscope image 27 is displayed in parallel with the 3D organ model 25. In the present embodiment, the rigid endoscope image is used as the medical image, but the medical image is not limited to this. For example, an ultrasound image can be used as the medical image.

The orientation matrix estimation unit 22 estimates an orientation matrix representing an orientation of the organ to be observed included in the rigid endoscope image in a three-dimensional coordinate system used for the 3D organ model. The estimation of the orientation matrix will be described in detail below. The display control unit 23 controls display of the 3D organ model based on the orientation matrix. Specifically, as shown in Fig. 2, the display control unit 23 controls display of the blood vessel inside the liver together with the display of the liver according to the orientation matrix. As a result, the display of the 3D organ model is updated in conjunction with the orientation of the organ to be observed, so that it is possible to reduce a burden on a doctor and an assistant and support anatomical understanding of a surgical field. This contributes to realizing high-quality surgery. The display control based on the orientation matrix will also be described in detail below. It is also considered to estimate a relative positional relationship between an imaging apparatus for capturing a medical image, such as an ultrasound probe, in addition to the rigid endoscope 11, and the organ to be observed as a display matrix representing the relative positional relationship in the three-dimensional coordinate system, and to control the display of the 3D organ model based on the display matrix. The estimation of the display matrix is performed by a display matrix estimation unit (not shown) executed by the processor. The relative positional relationship between the imaging apparatus and the organ to be observed is determined by, for example, the movement of the organ to be observed, the enlargement or reduction of the medical image, or the parallel movement.

In the present embodiment, as in JP2023-131741A, an imaging position is acquired, and an angle of the organ is estimated from the rigid endoscope image without estimating the angle of the organ. In addition, in JP2023-131741A, since a static imaging target is assumed, the imaging position is regarded as the orientation of the organ, and thus a dynamic imaging target in a dynamically changing rigid endoscope image as in the present embodiment is not assumed. In addition, as in Augmented Reality Navigation for Stereoscopic Laparoscopic Anatomical Hepatectomy of Primary Liver Cancer: Preliminary Experience (http:pubmed.ncbi.nlm.nih.gov/33842378), in a case in which 3D data is superimposed and displayed on a video during surgery, it is difficult to grasp a sense of depth, and an important structure may be hidden by superimposing and displaying the 3D data. However, in the present embodiment, since the 3D organ model 25 and the rigid endoscope image 27 are displayed in parallel, no structure is hidden. In addition, since a shape of the organ is deformed into a non-rigid body and separated, in a case in which the 3D data is superimposed and displayed on the video during the surgery as in Augmented Reality Navigation for Stereoscopic Laparoscopic Anatomical Hepatectomy of Primary Liver Cancer: Preliminary Experience (http:pubmed.ncbi.nlm.nih.gov/33842378), the shapes of the two are not matched, making it difficult to understand the correspondence relationship. On the other hand, in the case of the present embodiment, the 3D organ model 25 and the rigid endoscope image 27 are displayed in parallel from the beginning and the display of the 3D organ model is also updated in conjunction with the orientation of the organ to be observed, so that there is a low possibility that the relationship between the organ to be observed and the 3D organ model is difficult to understand.

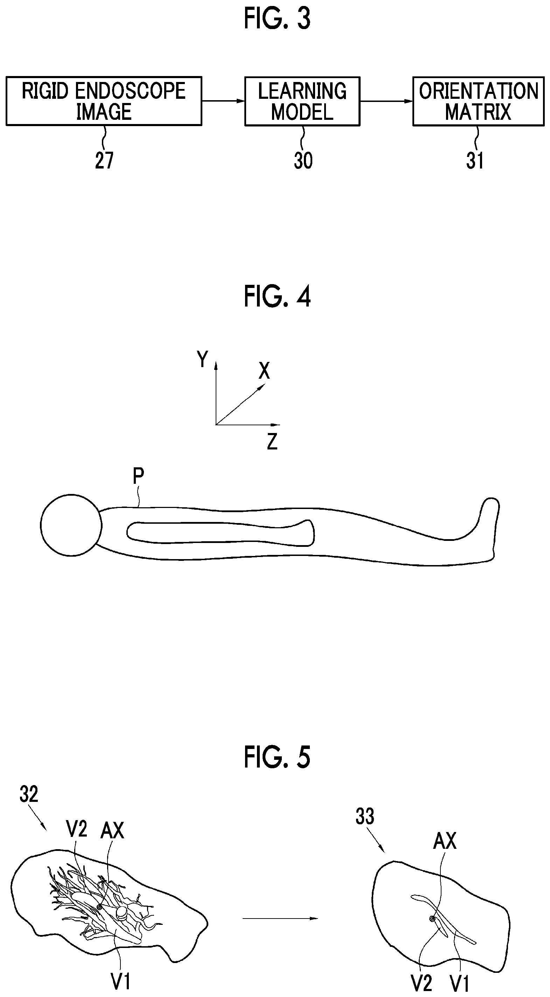

The estimation of the orientation matrix will be described below. As shown in Fig. 3, the rigid endoscope image and a learning model 30 that is trained using a correct orientation matrix for the rigid endoscope image as an input are used for the estimation of the orientation matrix. The rigid endoscope image actually obtained from the rigid endoscope 11 is input to the learning model, and thus an orientation matrix 31 is output from the learning model. The three-dimensional coordinate system representing the orientation matrix is represented by three axes of an X-axis, a Y-axis, and a Z-axis, as shown in Fig. 4. The X-axis is represented by a positive value that is zero on a right side of the patient P and that increases toward a left side. The Y-axis is represented by a positive value that is zero on a ventral side of the patient P and that increases toward a dorsal side. The Z-axis is represented by a positive value that is zero on a head side of the patient P and that increases toward a foot side. The three-dimensional coordinate system may be a polar coordinate system represented by a radius and a polar angle in addition to an orthogonal coordinate system such as an X-axis, a Y-axis, and a Z-axis, and is not particularly limited. In addition, instead of the learning model 30, a plurality of models such as a model that extracts a feature amount from the rigid endoscope image and a model that estimates the orientation matrix from the feature amount may be used to estimate the orientation matrix.

The display control based on the orientation matrix will be described below. As shown in Fig. 5, a 3D organ model 32 represents a model before the display control based on the orientation matrix, and a 3D organ model 33 represents a model after the display control based on the orientation matrix. Since the orientation matrix is estimated according to the orientation of the organ to be observed included in the rigid endoscope image, the display of the 3D organ model is also changed in accordance with the change in the orientation of the organ to be observed. Specifically, the display control unit 23 performs control of rotating the 3D organ model in the three-dimensional coordinate system. As a result, there is no longer a need to mentally compensate for a difference in an intraoperative appearance between the organ and the 3D organ model and to perform manual registration. A rotation axis AX can also be optionally set in addition to the orientation matrix. For example, a user's line of sight (a line connecting the centroid of the organ to be observed and the center of the field of view of the rigid endoscope) may be used as the rotation axis.

In the case of Fig. 5, in a case of checking how a blood vessel V2 runs under a thick blood vessel V1, the user moves a position of the rigid endoscope 11 to a position where the blood vessel V2 can be seen, thereby displaying a 3D organ model corresponding to the appearance of the organ in the rigid endoscope image, so that the user can check a running state of the blood vessel V2 under the thick blood vessel V1.

The display control unit 23 may periodically change the orientation of the 3D organ model in the three-dimensional coordinate system. In this case, the display control is performed in accordance with input from a periodic operation ON-OFF module, instead of the display control based on the orientation matrix. The input from the periodic operation ON-OFF module is performed using the user interface 16. Examples of the user interface 16 include input of the user, a gesture of the rigid endoscope 11, and voice recognition. In addition, automatic control such as turning ON the periodic operation after the rigid endoscope 11 is stationary for several seconds and turning OFF the periodic operation in a case in which the rigid endoscope 11 starts moving may be performed. In addition, it may be determined whether or not a designated structure is visible in a preoperative plan, and, in a case in which the designated structure is not visible, the periodic operation may be automatically activated. In addition, the periodic change in the orientation includes changes in up, down, left, and right directions in addition to a circular orbit with a line-of-sight direction as an axis. In addition, in a case in which a degree of overlap between structures in the organ to be observed is equal to or greater than a threshold value or in a case in which the orientation of the organ to be observed does not change for a certain period of time, the orientation of the 3D organ model may be periodically changed. A method described below can be used as a method of calculating the degree of overlap.

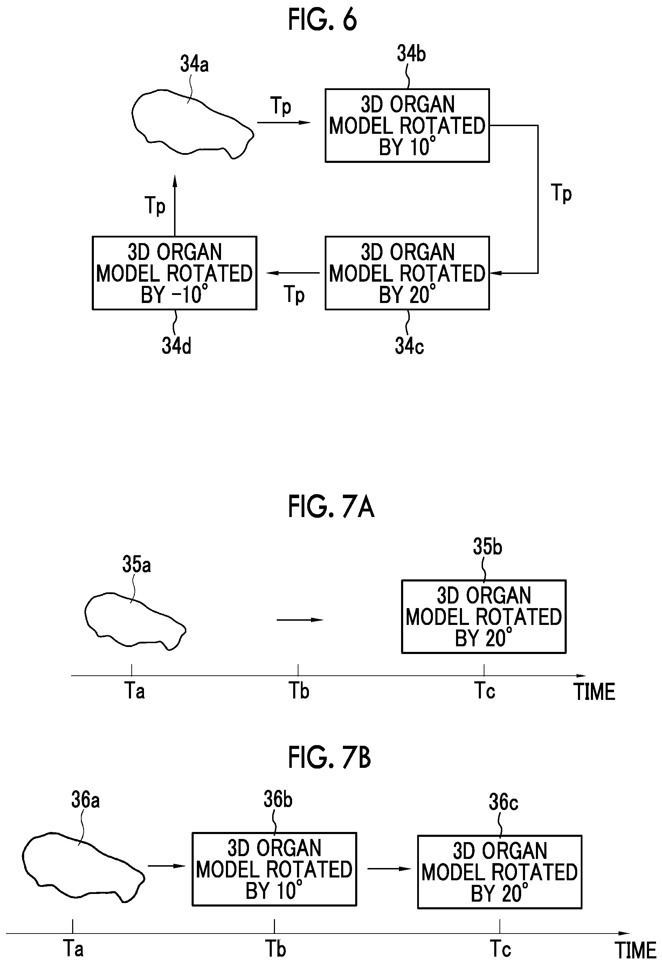

Specifically, as shown in Fig. 6, in a case in which a 3D organ model 34a is displayed at a predetermined angle and the periodic operation is set to ON by the periodic operation ON-OFF module, after a certain period of time Tp, a 3D organ model 34b obtained by rotating the 3D organ model 34a by 10° around the rotation axis is displayed. In addition, after a certain period of time Tp, a 3D organ model 34c obtained by rotating the 3D organ model 34b by 20° around the rotation axis is displayed. Then, after a certain period of time Tp, a 3D organ model 34d obtained by rotating the 3D organ model 34c by -10° around the rotation axis is displayed. In addition, after a certain period of time Tp, the 3D organ model 34d is rotated by -20° around the rotation axis, and thus the original 3D organ model 34a is displayed. By repeating the above, the orientation of the 3D organ model periodically changes. The periodic change may be a circular orbit.

In a case in which the display control is performed based on the orientation matrix, in order to prevent a sudden change in the orientation of the 3D organ model, it is also considered that the display control unit 23 performs a process of smoothing the change in angle of the 3D organ model over time on the orientation matrix. Specifically, in the case of Fig. 7A, through the display control based on the orientation matrix, display of a 3D organ model 35a at a timing Ta is updated to a 3D organ model 35b rotated by 20° at a timing Tc after the timing Ta. In this case, in order to prevent the change in angle of 20° of the 3D organ model, the orientation matrix is subjected to a process of smoothing the change in angle over time. In this case, as shown in Fig. 7B, a 3D organ model 36a at the timing Ta is updated to a 3D organ model 36b rotated by 10° at a timing Tb between the timing Ta and the timing Tc, and the 3D organ model 36a is updated to a 3D organ model 36c rotated by 20° at the timing Tc after the timing Tb. As a result, the change in angle of 20° of the 3D organ model can be gradually performed between the timings Ta to Tc.

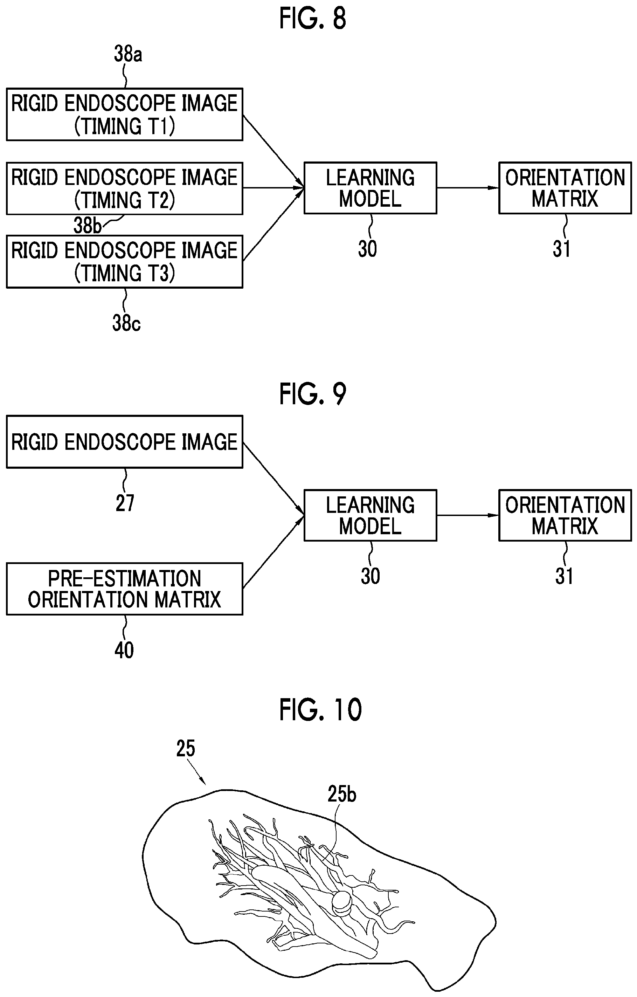

In order to improve the accuracy of the orientation estimation, the estimation of the orientation matrix may be performed using a plurality of rigid endoscope images obtained at different timings. Specifically, as shown in Fig. 8, a rigid endoscope image 38a obtained at a timing T1, a rigid endoscope image 38b obtained at a timing T2 different from the timing T1, and a rigid endoscope image 38c obtained at a timing T3 different from the timings T1 and T2 are input to the learning model 30, and the orientation matrix 31 is output. As a result, orientation estimation with higher accuracy can be expected for the movement of the 3D organ model that is updated in display in conjunction with the orientation of the organ to be observed, as compared with a case in which the estimation is performed only with the rigid endoscope image at a single timing.

In order to increase the accuracy of the orientation estimation, the estimation of the orientation matrix may be performed using the rigid endoscope image and the orientation matrix estimated before a timing at which the estimation is performed. Specifically, as shown in Fig. 9, the rigid endoscope image 27 and a pre-estimation orientation matrix 40 are input to the learning model 30. In response to this input, the orientation matrix 31 is output from the learning model 30. In a case in which the display control of the 3D organ model is performed based on the output orientation matrix, the orientation estimation with higher accuracy can be expected for the movement of the 3D organ model, as compared with a case in which the estimation is performed only with the rigid endoscope image at a single timing.

Regarding the display control based on the orientation matrix, the display control unit 23 may determine a degree of overlap between structures in the 3D organ model, and change the orientation of the 3D organ model such that the degree of overlap between structures falls within a specific range. As shown in Fig. 10, in the 3D organ model 25, a large number of the blood vessels 25b intersect with each other and are displayed to overlap each other. In a case in which the blood vessels overlap each other, it is difficult to understand how the blood vessels run, and thus it is also considered to display the 3D organ model in a way that minimizes the overlap of the blood vessels.

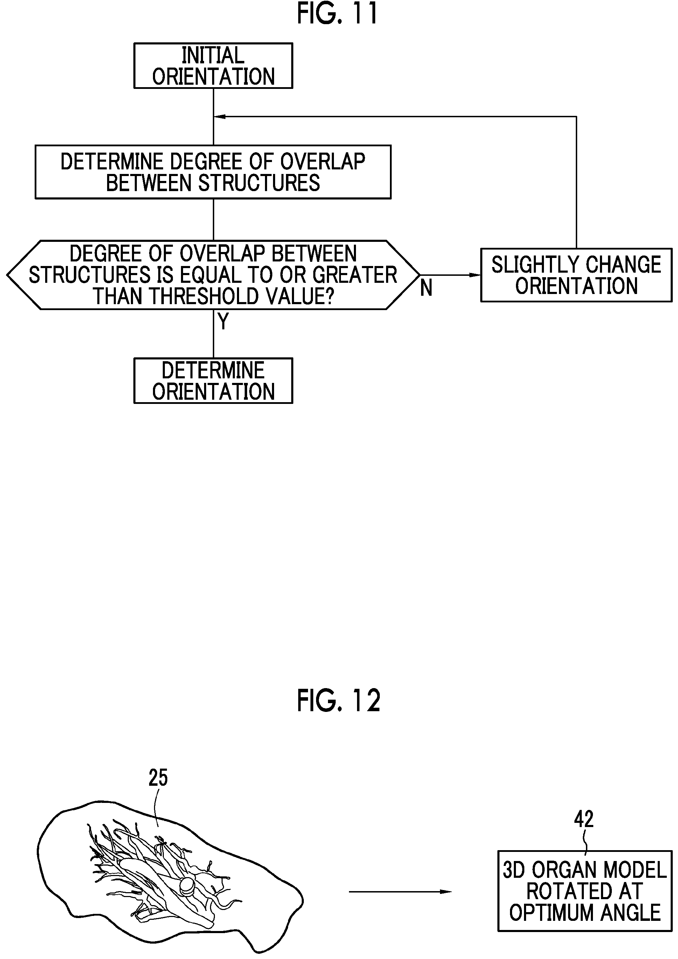

Therefore, as shown in Fig. 11, in a case in which an initial orientation of the 3D organ model is determined based on the orientation matrix, a degree of overlap between structures such as the blood vessels is determined from the 3D organ model of the initial orientation. In a case in which the degree of overlap is equal to or less than a threshold value, the 3D organ model is displayed based on the initial orientation. On the other hand, in a case in which the degree of overlap exceeds the threshold value, the degree of overlap between structures is determined again from the 3D organ model after slightly changing the initial orientation. This is repeated until the degree of overlap is equal to or less than the threshold value, and the 3D organ model is displayed based on an orientation in which the degree of overlap is equal to or less than the threshold value. As described above, by the orientation adjustment based on the degree of overlap between structures, a 3D organ model 42 obtained by rotating the 3D organ model 25 at an optimum angle at which the degree of overlap between structures is minimized can be displayed as shown in Fig. 12.

The determination of the degree of overlap between structures may be a determination of a degree of overlap between a specific structure designated by the user preoperatively or intraoperatively and another structure. In addition, in the determination of the degree of overlap between structures, the structures may be grouped, and, in a case of determining the degree of overlap between structures, the structures in the same group do not need to be determined as overlapping. In a case of grouping the structures, for example, in a case in which the structures are blood vessels, the structures may be grouped as separate structures for each branch of the blood vessel. The specific structure may be designated using the user interface 16.

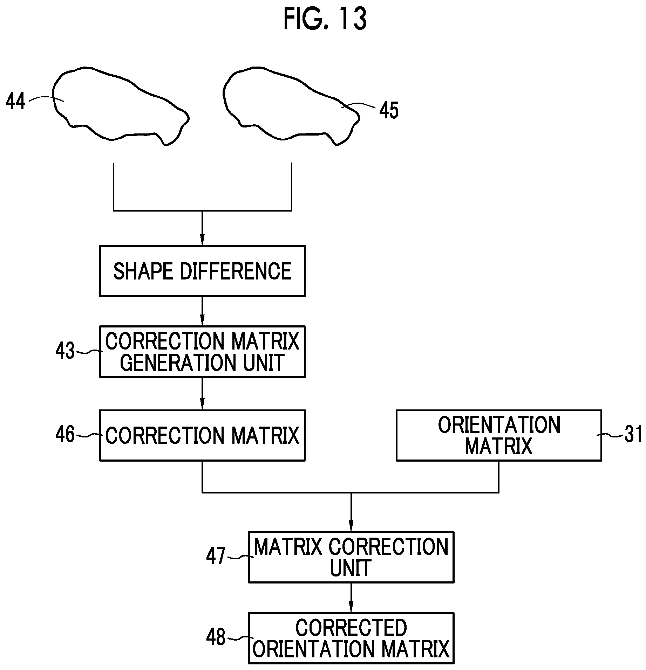

Since the 3D organ model that performs the display control based on the orientation matrix is extracted from a radiation image or an MRI image of the patient P, there is a difference in imaging conditions and a shape difference between patients. Therefore, as shown in Fig. 13, a correction matrix generation unit 43 calculates a correction matrix 46 for correcting a shape difference between a predetermined reference organ 44 and an organ to be observed 45. Then, a matrix correction unit 47 corrects the orientation matrix 31 in accordance with the correction matrix 46. The display control unit 23 controls the display of the 3D organ model based on a corrected orientation matrix 48 (corrected orientation matrix). As a result, it is possible to display the 3D organ model in which the shape difference is corrected. The correction matrix generation unit 43 and the matrix correction unit 47 are implemented by the central control unit including the processor executing the program in the program memory in the medical image processing device 12. In addition, in the present embodiment, the correction matrix 46 is used as the correction amount for correcting the shape difference between the reference organ 44 and the organ to be observed 45, but the orientation matrix 31 may be corrected using correction amounts such as yaw, pitch, and roll.

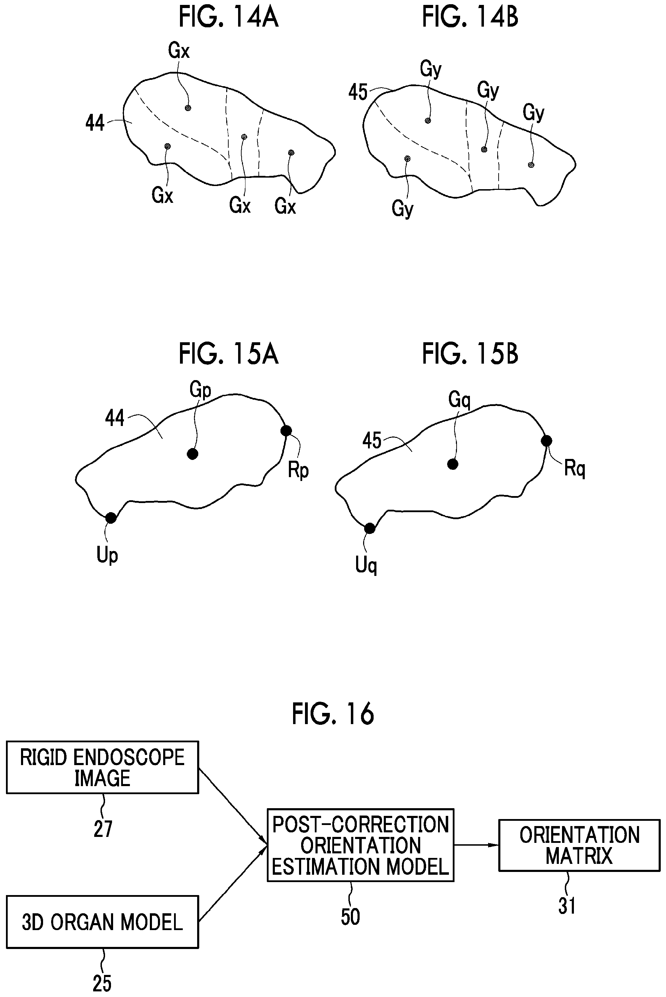

Specifically, as a method of calculating the correction matrix, as shown in Figs. 14A and 14B, linear registration is performed between a centroid Gx of each liver region of the reference organ 44, which is one of the reference organs, and a centroid Gy of each liver region of a liver to be observed 45, which is the organ to be observed, and the correction matrix is calculated from registration linear parameters. In addition, as shown in Figs. 15A and 15B, the correction matrix is calculated based on a positional relationship between a centroid Gp, a right end Rp, and a lower end Up in the reference organ 44 and a centroid Gq, the right end Rq, and the lower end Uq in the liver to be observed 45. As the method of calculating the correction matrix, in addition to the method of calculating the correction matrix based on a positional relationship such as a centroid, a method of using a correction model described below may be used, but the present invention is not limited to this, and other correction methods may be used.

In addition, instead of the correction matrix generation unit 43 and the matrix correction unit 47, as shown in Fig. 16, a post-correction orientation estimation model 50 may be used to calculate the orientation matrix 31 suitable for the patient. In this case, the post-correction orientation estimation model 50 estimates the orientation matrix 31 using the 3D organ model 25 in addition to the rigid endoscope image 27. By using the 3D organ model of the patient P who is a subject of the rigid endoscope image, the orientation matrix obtained by estimation is suitable for a shape of the patient and the like.

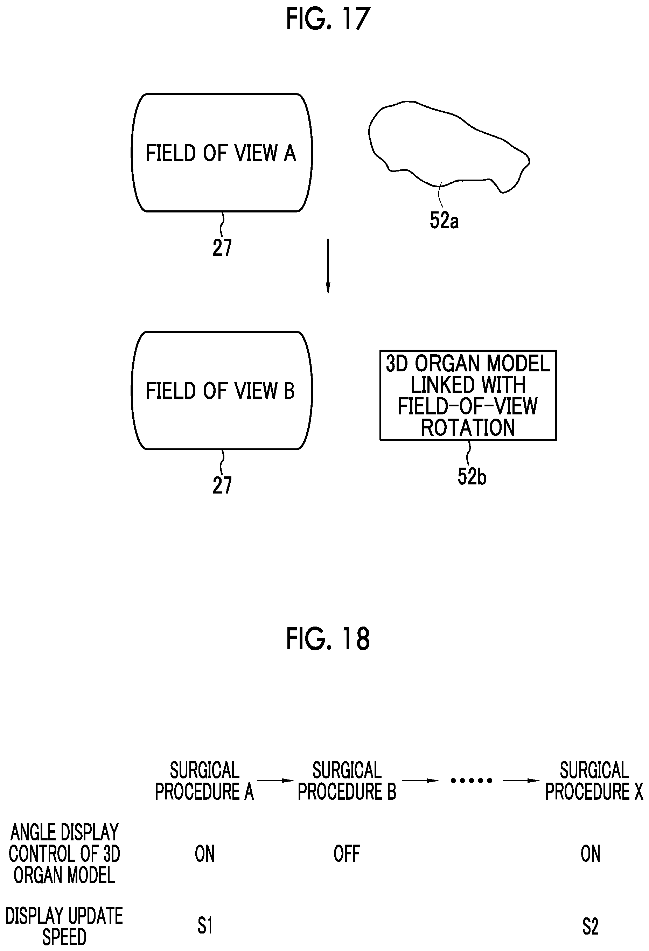

In the above-described embodiment, the display control unit 23 controls the display of the 3D organ model in conjunction with the orientation of the organ to be observed, but the display control unit 23 may perform the display control of the 3D organ model in conjunction with the field of view based on the rigid endoscope image. As shown in Fig. 17, in a case in which the rigid endoscope image 27 is displayed in a field of view A and a 3D organ model 52a is displayed at a specific angle, in a case in which the field of view A is changed to a field of view B, the display of the 3D organ model 52a is changed to a 3D organ model 52b that has been rotated, scaled, and/or translated corresponding to the movement from the field of view A to the field of view B.

In the above-described embodiment, a surgical procedure recognition unit may recognize a surgical procedure from the rigid endoscope image, and the display control unit 23 may turn ON or off angle display control of the 3D organ model or may control a display update speed according to the surgical procedure. As shown in Fig. 18, in a case in which the surgery is divided into a plurality of procedures such as a surgical procedure A, a surgical procedure B, ..., and a surgical procedure X, the angle display control of the 3D organ model is turned ON in the surgical procedure A and the surgical procedure X, and the display control of the 3D organ model is turned OFF in the surgical procedure B. In a case in which the angle display control of the 3D organ model is turned ON, the display control is performed based on the orientation matrix, and thus the angle control of the 3D organ model is performed in conjunction with the orientation of the organ to be observed. On the other hand, in a case in which the angle display control of the 3D organ model is turned OFF, the display control based on the orientation matrix is not performed, so that although the display of the 3D organ model is maintained, the angle control of the 3D organ model is not performed. The surgical procedure recognition unit is implemented by the central control unit including the processor executing the program in the program memory in the medical image processing device 12.

In addition, in a case in which the angle display control of the 3D organ model is turned ON, in the surgical procedure A, the display update speed of the 3D organ model is set to S1, while in the surgical procedure B, the display update speed of the 3D organ model is set to S2 different from S1. Since the surgical procedures are different from each other, the display update speed of the 3D organ model is changed according to the surgical procedures, so that the 3D organ model suitable for each surgical procedure can be displayed.

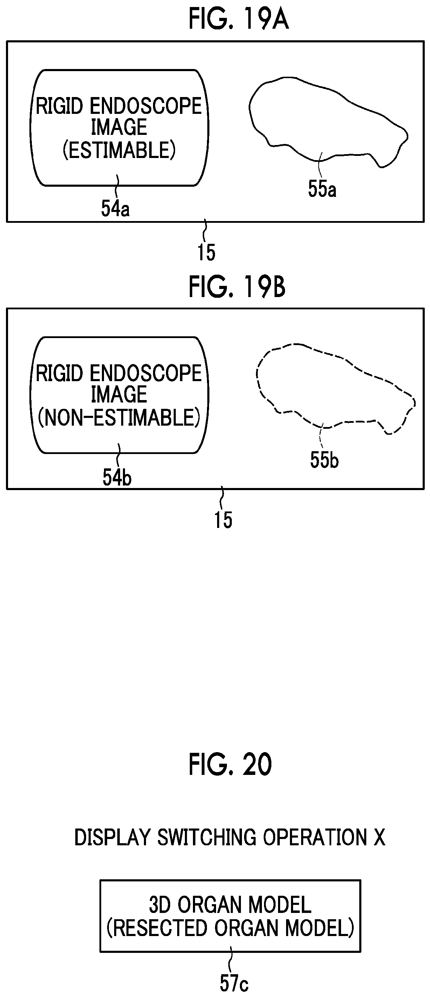

In the above-described embodiment, an estimation possibility determination unit (not shown) may determine whether or not the estimation of the orientation matrix works from the rigid endoscope image, and the display control unit 23 may stop the display of the 3D organ model in a case in which the estimation of the orientation matrix does not work. As shown in Fig. 19A, in a case in which the estimation possibility determination unit determines that the orientation matrix can be estimated from a rigid endoscope image 54a, the display control unit 23 executes display control of a 3D organ model 55a based on the orientation matrix. On the other hand, as shown in Fig. 19B, in a case in which the estimation possibility determination unit determines that the orientation matrix cannot be estimated from a rigid endoscope image 54b, the display control unit 23 stops display of a 3D organ model 55b (dotted line indicates that the display is stopped). The estimation possibility determination unit is implemented by the central control unit including the processor executing the program in the program memory in the medical image processing device 12. Note that images in which the estimation of the orientation matrix does not work include scenes in which the camera is too close to the organ or where smoke is present.

In the above-described embodiment, the angle display control of the 3D organ model is performed based on the orientation matrix, but instead of this, a display aspect of the 3D organ model may be switched in response to a display switching operation. The display switching operation is performed using the user interface 16. Specifically, as shown in Fig. 20, in a case in which a display switching operation X is performed, a resected organ model (for example, a resected liver model) created in advance in a preoperative resection simulation is displayed as a 3D organ model 57a.

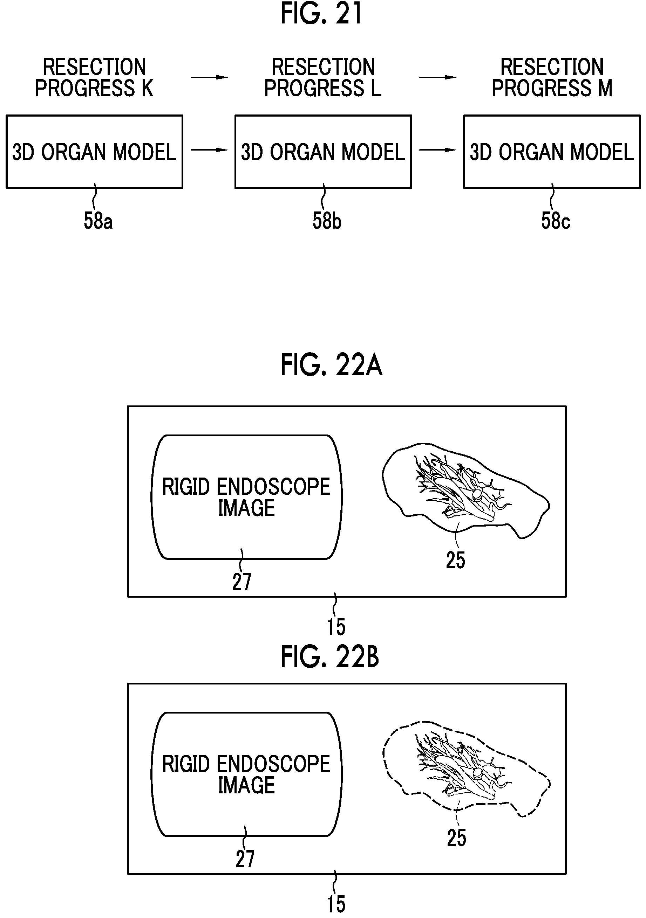

In the above-described embodiment, a progress recognition unit (not shown) may recognize a resection progress of the organ to be observed from the organ to be observed, and the display control unit 23 may switch the display aspect of the 3D organ model according to the resection progress. As shown in Fig. 21, in a case in which the resection progress is K, L, or M, the 3D organ models to be displayed are changed to different 3D organ models 58a, 58b, and 58c. For example, the 3D organ model 58a is a resected 3D organ model in which a specific cross section is resected. The progress recognition unit is implemented by the central control unit including the processor executing the program in the program memory in the medical image processing device 12.

In the above-described embodiment, in a case in which a movement recognition unit (not shown) recognizes a specific movement, the display control of the 3D organ model using the orientation matrix may be activated. It is also considered that the specific movement is a movement of the user, such as a gesture. In the case of Fig. 22A, the movement recognition unit recognizes a specific movement, and thus the display control of the 3D organ model 25 is activated (the activation state is represented by a solid line). Here, the activation of the display control of the 3D organ model 25 means that the display update of the 3D organ model 25 is executed. On the other hand, in the case of Fig. 22B, in a case in which the movement recognition unit does not recognize a specific motion, the display control of the 3D organ model 25 is stopped (the stopped state is represented by a dotted line). Here, the stop of the display control of the 3D organ model 25 means that the display update of the 3D organ model 25 is not executed in a state where the display of the 3D organ model 25 is maintained. In this case, angle rotation based on the orientation matrix or the like is not performed. The movement recognition unit is implemented by the central control unit including the processor executing the program in the program memory in the medical image processing device 12.

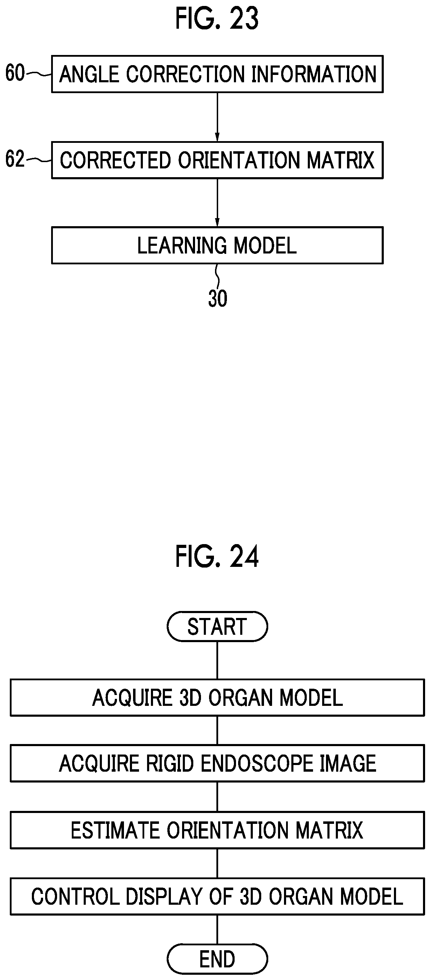

In the above-described embodiment, a correction reception unit may receive a correction from the user regarding a display direction of the 3D organ model, and correction information regarding the correction may be used for training the learning model or for a correction amount for correcting a shape difference between a predetermined reference organ and the organ to be observed. As shown in Fig. 23, the angle of the 3D organ model may be made rotatable using the user interface 16 even in a case in which the angle is controlled based on the orientation matrix. In this case, the rotation of the angle is recognized as angle correction information 60, and a corrected orientation matrix 62 is generated in accordance with the angle correction information 60. By using the corrected orientation matrix 62, the learning model 30 performs learning, so that the angle control of the 3D organ model can be performed more accurately. The angle correction information 60 may be used as the correction amount described above. Specifically, the correction amount may be reflected in the orientation matrix and may be used as the display matrix.

Next, the display control of the 3D organ model will be described with reference to a flowchart of Fig. 24. The 3D organ model acquisition unit 20 acquires a 3D organ model corresponding to the organ to be observed via the network NT or the like. The rigid endoscope image acquisition unit 21 acquires a rigid endoscope image obtained by the rigid endoscope 11 from the rigid endoscope 11. The rigid endoscope image and the 3D organ model are displayed on the display 15. The orientation matrix estimation unit 22 estimates an orientation matrix representing an orientation of the organ to be observed included in the rigid endoscope image in a three-dimensional coordinate system used for the 3D organ model. The display control unit 23 controls display of the 3D organ model based on the orientation matrix.

In the above-described embodiment, hardware structures of processing units that execute various types of processing, such as the 3D organ model acquisition unit 20, the rigid endoscope image acquisition unit 21, the orientation matrix estimation unit 22, the display control unit 23, the correction matrix generation unit 43, and the matrix correction unit 47, are various processors as described below. The various processors include a central processing unit (CPU) that is a general-purpose processor that executes software (programs) to function as various processing units, a graphical processing unit (GPU), a programmable logic device (PLD) that is a processor capable of changing a circuit configuration after manufacture, such as a field programmable gate array (FPGA), and an exclusive electric circuit that is a processor having a circuit configuration exclusively designed to execute various kinds of processing.

One processing unit may be configured of one of these various processors, or may be configured of a combination of two or more processors of the same type or different types (for example, a plurality of FPGAs, a combination of a CPU and an FPGA, or a combination of a CPU and a GPU). In addition, a plurality of processing units may be configured of one processor. As an example in which the plurality of processing units are configured of one processor, first, as typified by computers such as a client or a server, one processor is configured of a combination of one or more CPUs and software, and this processor functions as the plurality of processing units. Second, as typified by a system on chip (SoC) or the like, a processor that realizes the functions of the entire system including the plurality of processing units by using one integrated circuit (IC) chip is used. As described above, the various processing units are configured using one or more of the various processors as a hardware structure.

Further, the hardware structure of these various processors is more specifically an electric circuit (circuitry) in a form in which circuit elements such as semiconductor elements are combined. In addition, the hardware structure of the storage unit is a storage device such as a hard disc drive (HDD) or a solid state drive (SSD).

### Explanation of References

10: medical image processing system
11: rigid endoscope
12: medical image processing device
15: display
16: user interface
20: 3D organ model acquisition unit
21: rigid endoscope image acquisition unit
22: orientation matrix estimation unit
23: display control unit
25: 3D organ model
25a: liver
25b: blood vessel
27: rigid endoscope image
27a: liver
27b: structure
27c: ultrasound probe
30: learning model
31: orientation matrix
32, 33: 3D organ model
34a, 34b, 34c, 34d: 3D organ model
35a, 35b: 3D organ model
36a, 36b, 36c: 3D organ model
38a, 38b, 38c: rigid endoscope image
40: pre-estimation orientation matrix
42: (optimal angle rotation) 3D organ model
43: correction matrix generation unit
44: reference organ
45: organ to be observed
46: correction matrix
47: matrix correction unit
48: corrected orientation matrix
50: post-correction orientation estimation model
52a: 3D organ model
52b: (field-of-view-linked rotation) 3D organ model
54a: rigid endoscope image (estimable)
54b: rigid endoscope image (non-estimable)
55a, 55b: 3D organ model
57c: 3D organ model (resected organ model)
58a, 58b, 58c: 3D organ model
60: angle correction information
62: corrected orientation matrix
P: patient
AX: rotation axis
V1, V2: blood vessel
Gx, Gy: centroid
Gp, Gq: centroid
Rp, Rq: right end
Up, Uq: lower end

## Claims

1. A medical image processing device comprising:
a processor (12),
wherein the processor is configured to
acquire a 3D organ model (52a) corresponding to an organ to be observed (45), which is an observation target,
acquire a medical image including the organ to be observed,
estimate an orientation matrix (48) representing an orientation of the organ to be observed included in the medical image in a three-dimensional coordinate system used for the 3D organ model, and
control display of the 3D organ model based on the orientation matrix.

2. The medical image processing device according to claim 1,
wherein the estimation of the orientation matrix is performed using the medical image and a learning model that is trained with the medical image and its corresponding correct orientation matrix as an input.

3. The medical image processing device according to claim 1,
wherein the processor is configured to perform control of rotating the 3D organ model in the three-dimensional coordinate system.

4. The medical image processing device according to claim 1,
wherein the processor is configured to periodically change an orientation of the 3D organ model in the three-dimensional coordinate system in a case in which a degree of overlap between structures in the organ to be observed is equal to or greater than a threshold value or in a case in which the orientation of the organ to be observed does not change for a certain period of time.

5. The medical image processing device according to claim 1,
wherein the processor is configured to determine a degree of overlap between structures in the 3D organ model, and change an orientation of the 3D organ model such that the degree of overlap between structures falls within a specific range.

6. The medical image processing device according to claim 1,
wherein the processor is configured to
calculate a correction amount for correcting a shape difference between a predetermined reference organ and the organ to be observed and correct the orientation matrix according to the correction amount, and
control the display of the 3D organ model based on the corrected orientation matrix.

7. The medical image processing device according to claim 1,
wherein the processor is configured to estimate the orientation matrix using the 3D organ model in addition to the medical image.

8. The medical image processing device according to claim 1,
wherein the processor is configured to apply a process of smoothing a change in angle of the 3D organ model in a time direction to the orientation matrix.

9. The medical image processing device according to claim 1,
wherein the estimation of the orientation matrix is performed using a plurality of the medical images obtained at different timings.

10. The medical image processing device according to claim 1,
wherein the estimation of the orientation matrix is performed using the medical image and an orientation matrix estimated before a timing at which the current estimation is performed.

11. The medical image processing device according to claim 1,
wherein the display of the 3D organ model is controlled in conjunction with a field of view based on the medical image.

12. The medical image processing device according to claim 1,
wherein the processor is configured to recognize a surgical procedure from the medical image, and according to the surgical procedure, to turn on or off of display control of an angle of the 3D organ model or to adjust a display update speed.

13. The medical image processing device according to claim 1,
wherein the processor is configured to determine whether or not the estimation of the orientation matrix works from the medical image, and stop the display of the 3D organ model in a case in which the estimation of the orientation matrix does not work.

14. The medical image processing device according to claim 1,
wherein the processor is configured to switch a display aspect of the 3D organ model in response to a display switching operation.

15. The medical image processing device according to claim 1,
wherein the processor is configured to recognize a resection progress of the organ to be observed from the organ to be observed, and switch a display aspect of the 3D organ model according to the resection progress.

16. The medical image processing device according to claim 1,
wherein the processor is configured to, in a case in which a specific movement is recognized, activate display control of the 3D organ model using the orientation matrix.

17. The medical image processing device according to claim 2,
wherein the processor is configured to receive a user correction regarding a display direction of the 3D organ model, and correction information regarding the user correction is used for training the learning model or for determining a correction amount for correcting a shape difference between a predetermined reference organ and the organ to be observed.

18. The medical image processing device according to any one of claims 1 to 17,
wherein the medical image is any one of a rigid endoscope image or an ultrasound image.

19. The medical image processing device according to any one of claims 1 to 17,
wherein the 3D organ model is extracted from a radiation image or an MRI image.

20. The medical image processing device according to any one of claims 1 to 17,
wherein the organ to be observed is any one of a liver, a kidney, a pancreas, a uterus, or a nerve.

21. The medical image processing device according to any one of claims 1 to 17,
wherein the processor is configured to estimate a relative positional relationship between an imaging apparatus for capturing the medical image and the organ to be observed as a display matrix representing the relative positional relationship in the three-dimensional coordinate system, and control display of the 3D organ model based on the display matrix.

22. A method of operating a medical image processing device, the method comprising steps, performed by a processor, of:
acquiring a 3D organ model (52a) corresponding to an organ to be observed (45), which is an observation target;
acquiring a medical image including the organ to be observed;
estimating an orientation matrix (48) representing an orientation of the organ to be observed included in the medical image in a three-dimensional coordinate system used for the 3D organ model; and
controlling display of the 3D organ model based on the orientation matrix.
